# EUROPÄISCHE PATENTANMELDUNG

(11) **EP 1 230 896 A2**
(43) Veröffentlichungstag der Anmeldung: **14.08.2002**
(21) Anmeldenummer: 02001672.1
(22) Anmeldetag: 24.01.2002
(51) Int. Cl.: A61B 6/14

(54) **Verstellbares Halter-System zur Positionierung eines Aufnahmemediums bei Röntgenaufnahmen**

(30) Priorität: 27.01.2001 DE 20100641 U; 17.03.2001 DE 10113092
(71) Anmelder: Herzog, Rainer, 90537 Feucht (DE); Steer, Sebastian, Dr., 84137 Vilsbiburg (DE)
(72) Erfinder: Herzog, Rainer, 90537 Feucht (DE); Steer, Sebastian, Dr., 84137 Vilsbiburg (DE)

(57) **Zusammenfassung**

Die Erfindung betrifft ein Halter-System, dass im wesentlichen eine einzige universelle mechanische Vorrichtung zur Abdeckung aller möglichen Positionierungs -Varianten für ein röntgenempfindliches Aufnahmemedium (3) aufweist und im wesentlichen einteilig ausgebildet ist.
Mit einem derartigen Halter-System lassen sich alle Positionseinstellungen ohne Wechsel von Verbindungsteilen/Stangen etc. problemlos durchführen. Insbesondere Sensoren können bei verschiedenen aufeinanderfolgenden Aufnahmen im Halter-System fixiert bleiben.
Die hygienischen Bedingungen werden erweitert indem zum einen eine komplette Abdeckung von Aufnahmemedium und Halter-System geschieht zum andern weniger Schutzhüllen benötigt werden.
Ergänzungen des Halter-Systems um einen Mechanismus zur Fixierung sowie einer Verschiebung entlang des Objektes erweitern ein einfaches Röntgensystem um die Möglichkeit exakte Winkeldarstellungen sowie Schichtaufnahmen dieses Objektes durchzuführen.

Bei konventionellen Systemen der Positionierung respektive Halterung des Aufnahmemediums geschieht dies durch Systeme, die sich aus mehreren Teilen zusammensetzen. Diese Teile (verschieden gebogene Stangen, Plastikverbinder und Adapter) werden je nach Notwendigkeit der Positionierung zusammengesteckt und oder verbunden und das Aufnahmemedium daran befestigt.

Der Erfindung liegt die Aufgabe zugrunde, ein Halter-System zu realisieren, das eine einfache und schnelle Positionierung eines Aufnahmemediums ohne Wechsel und aufwendigem Umbau von Halterungen ermöglicht.

## Beschreibung

Die Erfindung betrifft ein Halter-System zur einfachen Positionierung von Aufnahmemedien, wie Röntgenfilmen, Folien und oder Sensoren, im Bezug zum Röntgengenerator. Beispielsweise findet das Halter-System Verwendung, um das Aufnahmemedium in Lage und Position zum Röntgenstrahler durch einfache Handhabung zu positionieren. Das Halter-System kann im wesentlichen auf allen radiologischen Gebieten zum Einsatz kommen.

Ein derartiges Halter-System findet insbesondere Anwendung in der Zahnmedizin zur Positionierung eines Aufnahmemediums (Röntgenfilmen, Sensoren etc.) im Mund. Unter Verwendung der Paralell-, Halbwinkel- und Rechtwinkel - Technik werden hier mit einem einzigen Halter-System alle möglichen Positionen, beispielsweise Bissflügelaufnahmen (Quer- und Hochformat), Frontaufnahmen, Aufnahmen in allen vier Quadranten, Aufbisse, Weißheitszahnaufnahmen etc., erfasst.

Bei der konventionellen Art der Positionierung respektive Halterung des Aufnahmemediums geschieht dies durch Systeme, die sich aus mehreren Teilen zusammensetzen. Diese Teile (verschieden gebogene Stangen, Plastikverbinder und Adapter) werden je nach Notwendigkeit der Positionierung zusammengesteckt und oder verbunden und das Aufnahmemedium daran befestigt. Der Anwender muß oft lange überlegen, welche Teile für die verschiedenen Positionierungen, wie zusammengefügt werden müssen. Bei der Rechtwinkeltechnik ist das Aufnahmemedium dabei starr mit dem Tubus des Röntgengerätes verbunden, bei der Parallelaufnahmetechnik wird eine separate Verbindung mit einem Visierring und Aufbissstück verwendet.

Die konventionelle Art benötigt dabei zur intraoralen Positionierung mindestens 2 oder mehr Stangen und/ oder Adapter.
Die Vielzahl der dazu notwendigen Einzelteile führt auf Grund der Unübersichtlichkeit zu erheblichen Anwendungsproblemen in der Praxis.

Der Erfindung liegt die Aufgabe zugrunde, ein Halter-System der eingangs erwähnten Art zu realisieren, das eine einfache und schnelle Positionierung eines Aufnahmemediums ohne Wechsel und aufwendigem Umbau von Halterungen ermöglicht. Insbesondere soll dieses Halter-System möglichst aus einer mechanischen Vorrichtung/Komponente bestehen.

Diese Aufgabe wird durch ein Halter-System der Eingangs genannten Art dadurch gelöst, dass das Halter-System im wesentlichen eine einzige universelle mechanische Vorrichtung zur Abdeckung aller möglichen Positionierungs -Varianten für ein röntgenempfindliches Aufnahmemedium aufweist.

Die Erfindung geht dabei von der Erkenntnis aus, dass es in der praktischen Radiologie, beispielsweise bei Röntgenaufnahmen im Dentalbereich oft sehr schwer ist, sowohl in Paralleltechnik als auch in Rechtwinkeltechnik Aufnahmen zu erstellen. Der Grund hierfür liegt darin, dass die jeweiligen Aufnahmetechniken nur schwierig mit den vorhandenen Hilfsmitteln umzusetzen sind.

Ein Halter-System, das generell eine zu handhabende mechanische Komponente darstellt, ist in der Praxis wesentlich einfacher und leichter zu handhaben als herkömmliche Systeme. Im wesentlichen existiert nur noch ein einziges mechanisches Teil, das zur intraoralen Positionierung dient.

Beispielsweise wird durch einfaches Drehen oder Verschieben des Aufnahmemediums die Lage verändert und ermöglicht dem Arzt oder der Röntgenassistentin dadurch eine unkomplizierte Positionierung.

Verschiedene Halteklammem bieten die Möglichkeit, Aufnahmemedien verschiedener Größe und Hersteller aufzusetzen.
Innerhalb einer spezifischen Anwendung finden dabei meist nur Aufnahmemedien eines Herstellers Anwendung.

Das Strahlen- Nutzfeld kann durch Anwendung eines solchen Halter-Systems für alle Positionierungen auf einfache Weise reduziert werden, dadurch ergibt sich eine geringere Strahlenbelastung für den Patienten. Beispielsweise auf ein kreisförmiges Feld mit Radius r (tubusabhängig).
Eine weitere Reduktion der Strahlenbelastung ist durch eine , der Größe und Form des Aufnahmemediums angepasste Blende möglich.
Diese befindet sich am Tubus oder Visierring und wird jeweils auf die gleiche Rotationsstellung wie das Aufnahmemedium gebracht.

Mit einem Halter-System der eingangs erwähnten Art lassen sich alle Positionseinstellungen ohne Wechsel von Verbindungsteilen/Stangen etc. problemlos durchführen. Insbesondere Sensoren können bei verschiedenen aufeinanderfolgenden Aufnahmen im Halter-System fixiert bleiben.

Die aus hygienischen Gründen nötigen Schutzhüllen bei Sensoren können bei allen Haltereinstellungen (Rotation oder Translation) belassen werden. Pro Patient ist nur eine Schutzhülle nötig, ein Sterilisieren oder Desinfizieren des Halter-Systems ist bei einer entsprechenden Schutzhülle nicht erforderlich. Es sind die üblichen Desinfektionsmassnahmen einzuhalten.

Das Halter-System kann über die Grundpositionierungs -Varianten hinaus eingestellt werden. Insbesondere können alle möglichen Zwischenpositionen individuell den topographischen Gegebenheiten, beispielsweise im Weisheitszahnbereich, angepasst werden.

Bissflügelaufnahmen können je nach Zahnbogenkrümmung und Platzangebot mit hoch- oder querstehenden Format durchgeführt werden.

Da meistens pro Seite zwei Aufnahmen erforderlich sind, kann die mittig stehende Haltestange für die hintere Aufnahme (Zahnnummern:6,7,8) mesial (von vorne), für die vordere Aufnahme (Zahnnummern: 5,4,3) distal (von hinten) des Aufnahmemediums durch die Zahnreihen geführt werden. Der Querschnitt der Haltestange sollte im Bereich der Zahnreihen-Durchführung möglichst klein gehalten werden.

Bei Messaufnahmen mit Kanalinstrumenten (Endodontie) ist mit dem Einsatz des Halter-Systems die Positionierung einfacher zu handhaben.
Durch Ergänzung des Halter-Systems um eine Fixierung am Aufnahmeobjekt beispielsweise von einem Zahn können davon durch Drehen des Halter-Systems um diesen Fixpunkt Aufnahmen aus verschiedenen Winkeln gemacht werden.

Eine weitere Ergänzung des Halter-Systems ermöglicht es das Halter-System mit Aufnahmeemedium zur Schichtaufnahme eines Objektes herzunehmen. Hier wird zusätzlich zu der punktuellen Fixierung noch eine Verschiebung des Aufnahmemediums bewirkt. Die Verschiebung geschieht entlang des Objektes und muß auf Grund der Geometrie nicht notwendigerweise direkt am Aufnahmemedium erfolgen. Die Verschiebung entlang des Objektes und gleichzeitige Rotationsbewegung um diesen verschiebbaren Punkt bilden die Grundlage für eine Schichtaufnahme.
Diese Art der Schichtaufnahme kommt mit einer einfachen Primärschlitzblende aus, kann mit einem kostengünstigen Strahler durchgeführt werden und erfordert keine wesentlichen Anschaffungen bezüglich eines Schichtaufnahmegerätes.
Im folgenden wird das Halter-System anhand der in den Figuren dargestellten Ausführungsbeispielen näher beschrieben und erläutert. Es zeigen:
**Figur 1** ein erstes Ausführungsbeispiel der Darstellung des Halter-Systems.
**Figur 2** ein zweites Ausführungsbeispiel der Aufsicht auf das Halter-System, den Haltermechanismus mit eingezeichnetem Strahlenfeld.
**Figur 3a** ein weiteres Ausführungsbeispiel der Befestigung des Halter-Systems am Tubus des Strahlers.
**Figur 3b** ein weiteres Ausführungsbeispiel der Befestigung des Halter-Systems am Visierring.
**Figur 4** ein weiteres Ausführungsbeispiel des Halter-Systems in Verbindung mit dem Visierring als Federring und der Strahlenblende.
**Figur 5a** ein weiteres Ausführungsbeispiel der Strahlenfeldbegrenzung ohne Blende (Maske) durch Anklemmen am Tubus.
**Figur 5b** ein weiteres Ausführungsbeispiel der Feldbegrenzung mit Blende.
**Figur 6** ein weiteres Ausführungsbeispiel der Prinzis einer Röntgenaufnahme am Beispiel der Einstellung einer intraoralen Bissflügelaufnahme.
**Figur 7a** ein weiteres Ausführungsbeispiel des anatomischen Aufnahmeschema am Beispiele eines Schemenbildes der intraoralen Grund Positionierungs Varianten.
**Figur 7b** ein weiteres Ausführungsbeispiel eines anatomischen Aufnahmeschematas am Beispiel von intraoralen Grund Positionierungs Varianten.
**Figur 8a-c** ein weiteres Ausführungsbeispiel der radiologischen Abbildungstechniken insbesondere in der Zahnmedizin (R-; P- und H-Winkel-Technik).
**Figur 9** ein weiteres Ausführungsbeispiel mit der Besonderheit der direkten Integration eines wiederverwendbaren Aufnahmemediums
**Figur 10** ein weiteres Ausführungsbeispiel für Aufnahmen des gleichen Objektes aus verschiedenen Winkel
**Figur 11** ein weiteres Ausführungsbeispiel für die Verwendung des Halter-Systems als Schichtaufnahme - Gerät
**Figur 12** ein weiteres Ausführungsbeispiel zur Verdeutlichung der Bewegungsabläufe bei der Schichtaufnahmetechnik.

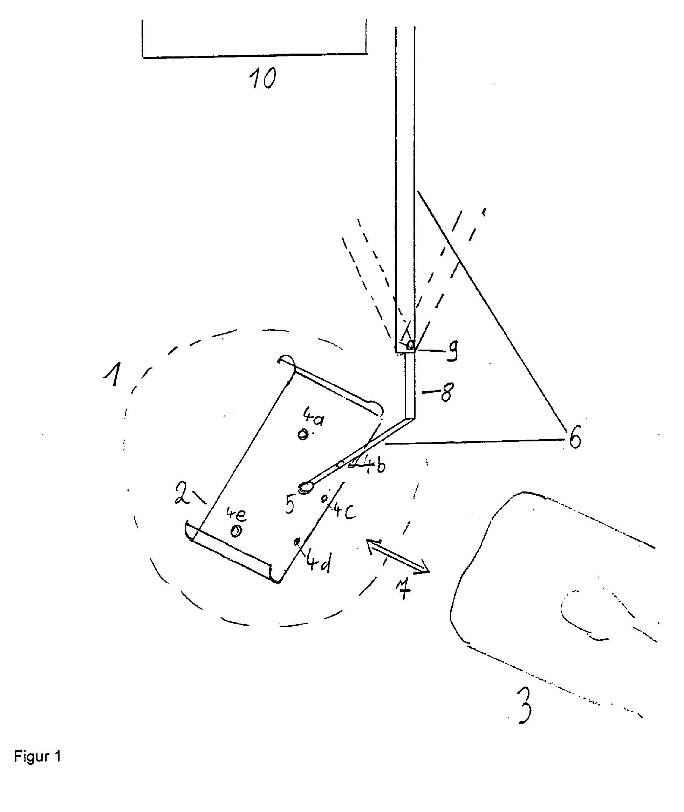

**Figur 1:** Das Halter-System umfasst einen Haltemechanismus 1 mit einer Halteklammer 2, die universell ausgeführt ist (Anpassungen der Klammer an das Aufnahmemedium), einem Rotationspunkt 5 sowie einer daran befestigten Haltestange 6, die zur Befestigung des Haltemechanismus 1 am Tubus der Röntgenröhre 10 dient.
Das röntgenempfindliche Aufnahmemedium 3, beispielesweise Sensor,Film oder Folie wird in der Halteklammer 2 fixiert.
In der Halteklammer 2 kann das Aufnahmemedium 3 verschoben 7 und oder die Halteklammer mit dem Aufnahmemedium verschoben werden.
Die Halteklammer 2 ist je nach Art des verwendeten Aufnahmemediums verschieden ausgebildet.
Der Punkt 5 ist Rotationspunkt von dem aus die Haltestange 6 nach einem Winkel von beispielsweise 90° zum Tubus 10 der Rötgenröhre führt.
Die Fixierpunkte 4a-e, die um den Punkt 5 angeordnet sind, dienen zur Einstellung der verschiedenen Grund-Positionierungsmöglichkeiten.
Die Haltestange ist im Bereich der Zahnreihendurchführung 8 extrem dünn ausgebildet. Der Punkt 9 der Haltestange 6 ist so ausgelegt, dass dieser durch ein Gelenk beweglich gestaltet sein kann.
Das Abwinkeln 9 der Haltestange 6 in sensomahem Bereich dient dazu, um auch die Halbwinkeltechnik mit dem Halter-System durchführen zu können. Vom Punkt 9 führt die Haltestange 6 zum Tubus 10.

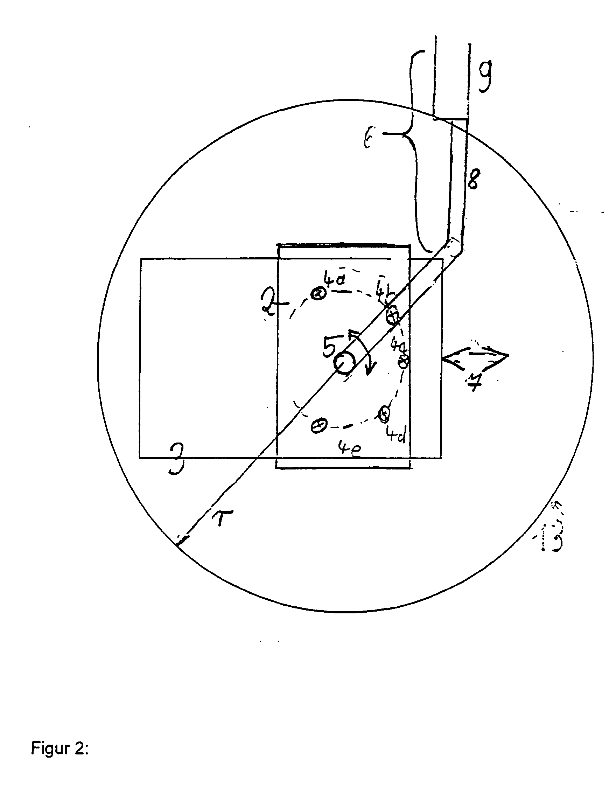

**Figur 2** zeigt im wesentlichen die in Figur 1 beschriebenen Komponenten. Der Rotationspunkt 5, sowie die Rasterpunkte 4a-e sind als Draufsicht dargestellt.
Des weiteren befindet sich das Aufnahmemedium 3 innerhalb der Halteklammer 2. Die Translation 7 und der Rotationpunkt 5 sind hervorgehoben dargestellt.
Die verschiedenen Grund- Positionierungsmöglichkeiten entstehen durch die Rotation 5 und die Fixierung an den Rasterpunkten 4a-e.
Zwischen den Rasterpunkten 4a-e sind alle Positionen möglich.

Der Kreis 13 um den Haltemechanismus stellt den Bereich dar, wo prinzipiell während des Röntgens Strahlung existiert.

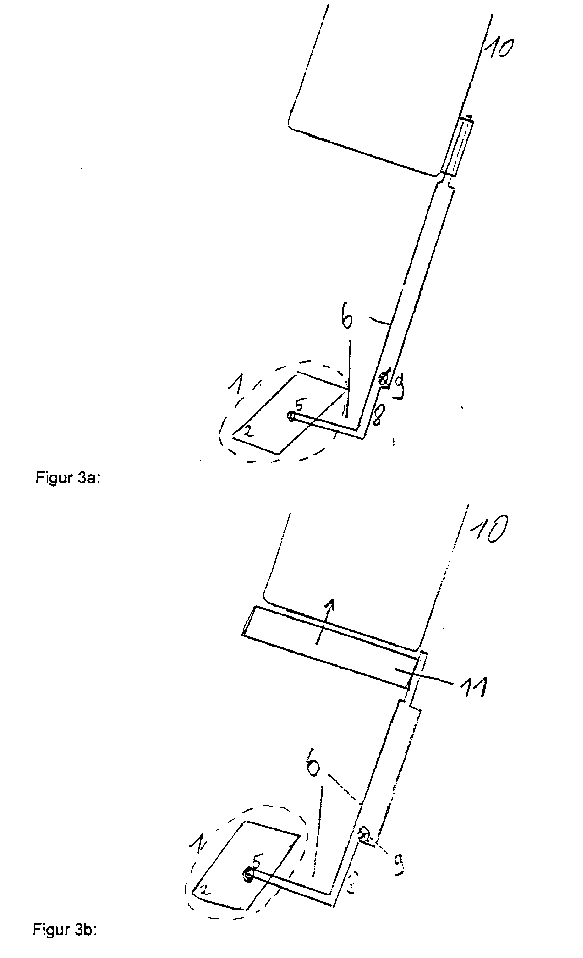

**Figur 3a und 3b** stellen die Fixierung des Halter-Systems direkt am Tubus der Röntgenröhre oder am Visierring dar.
Dabei ist der Haltemechanismus 1 vereinfacht dargestellt. Die Haltestange 6 mit der Verjüngung 8 und dem möglichen Gelenk 9 ist in Figur 3a direkt am Tubus/Röhre 10 fixiert.
In Figur 3b dient ein Visiering 11 zur Aufnahme der Haltestange 6.

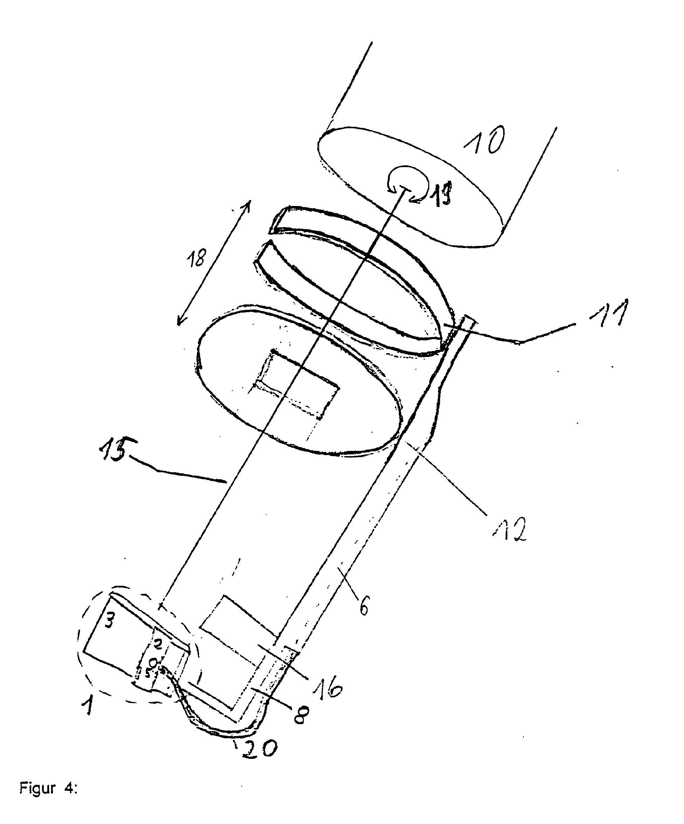

**Figur 4** zeigt das Halter-System in Verbindung mit dem Visierring 11 und einer Strahlenblende 12.
Der Visierring 11 kann dabei als Federring (bei Bedarf mit einem Adapter an den Tubus/Strahler) ausgeführt sein, der gleichzeitig herstellerunab-hängig auf jeden Tubus innen oder außen aufgeklemmt oder eingespreizt werden kann.
Gleichzeitig ist die notwendige Rotation 19 des Halter-Systems um den Zentralstrahl 15 gewährleistet. Der Visierring 11 ist drehbar am Tubus /Strahler 10 angebracht.
An der Haltestange 6 kann sich eine entsprechende Blende 12 befinden.
Die Blende 12 kann, da beweglich auf der Haltestange 6 angebracht, ebenso zur Fixierung des Abstandes zwischen Aufnahmemedium und Röntgenstrahler ausgebildet sein.
Dabei ist die Blende 12 so ausgelegt, dass Strahlung nur auf das Aufnahmemedium trifft.
Weitgehendst ist Streustrahlung und belastende Strahlung für den Patienten durch die besondere Form der Blende ausgeschlossen.
Im Bereich nahe des Aufnahmemediums ist der Querschnitt 8 der Haltestange 6 zur besseren Durchführung durch die Zahnreihen bei Bissflügelaufnahmen wesentlich reduziert.
Wird das Halter-System als separates Halter-System mit Visierring 11 verwendet ,so ist zur Lagestabilisierung ein entsprechendes Kunststoffbeissstück 16 vorgesehen.
Der Abstand der Röntgenquelle (Tubus) zum Aufnahmemedium kann durch verschieben 18 des Halter-Systems verändert werden, wodurch die Doisisleistung am Aufnahmemedium verändert wird .
Bei der Verwendung eines Sensors 3 wird das notwendige Kabel 20 direkt an der Haltestange 6 geführt.

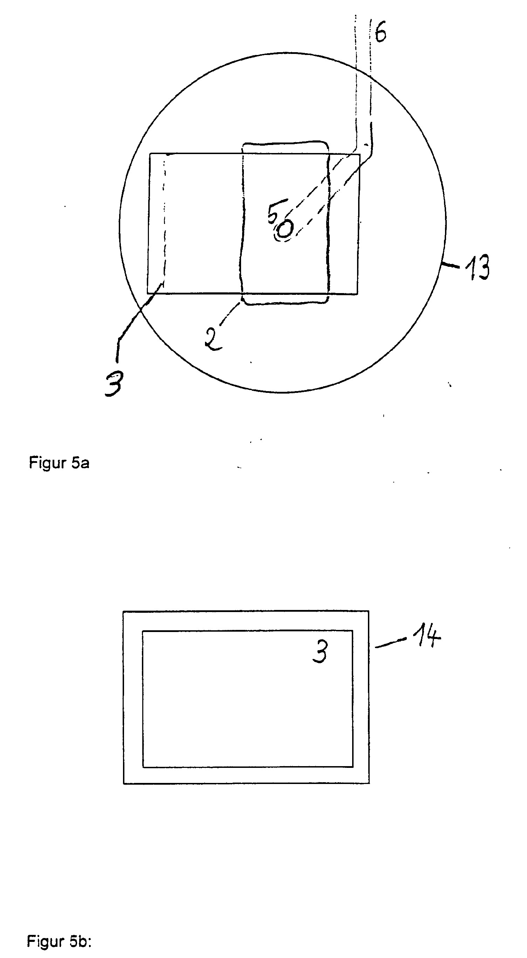

**Figur 5a und 5b** zeigen die Strahlenfeldbegrenzung. Dabei zeigt 5a die Strahlenfeldbegrenzung ohne Blende 13 . Figur 5b stellt die Begrenzung 14 durch die Blende, die entsprechend des Aufnahmemediums ausgebildet ist, dar. Die Blende 12 ist dabei auf die geometrische Lage des Aufnahmemediums eingestellt.

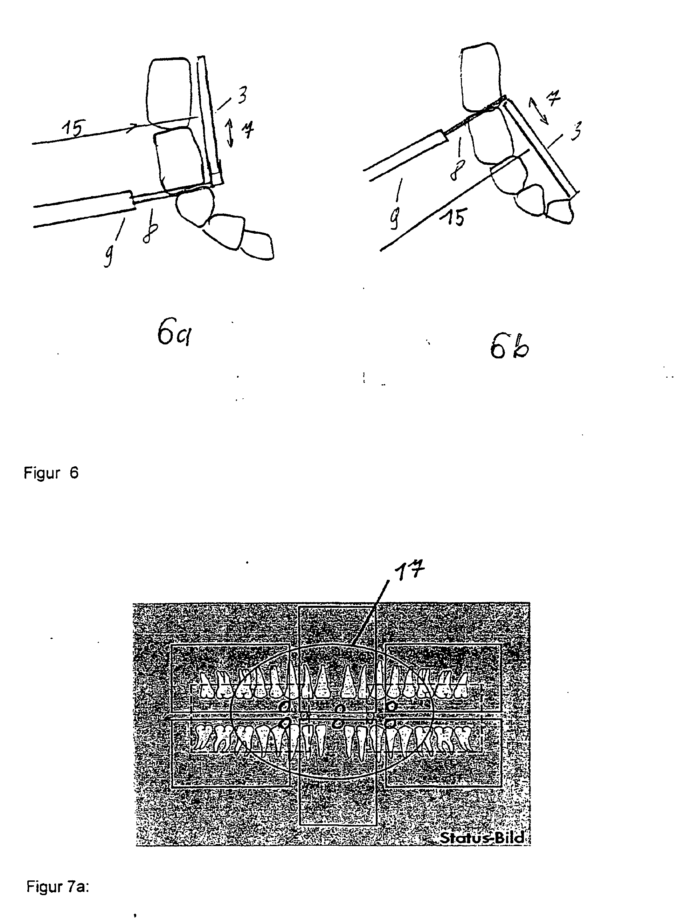

**Figur 6a und 6b** zeigen, dass beispielsweise in der Zahnmedizin die Anwendung des Halter-Systems durch einfaches Rotieren des Halter-Systems 19 um den Zentralstrahl 15, hier 180°, und oder Verschieben 7, auch an schwierig zugänglichen Stellen, eine schnelle Handhabung ermöglicht.

**Figur 7a** zeigt am Beispiel intraoraler Grundpositionierungs Varianten die verschiedenen Möglichkeiten der Positionierung.
Es wird die Problematik aufgezeigt, dass durch ein Fenster 17 alle Positionierungen erreicht werden müssen, die Bissflügelaufnahmen (Querund Hochformat), Frontaufnahmen, Aufnahmen in alle vier Quadtanten, Aufbisse, Weißheitszahnaufnahmen etc. umfassen.
Bei den anderen radiologischen Anwendungsfällen gestalten sich die Aufnahmesituationen ähnlich komplex.

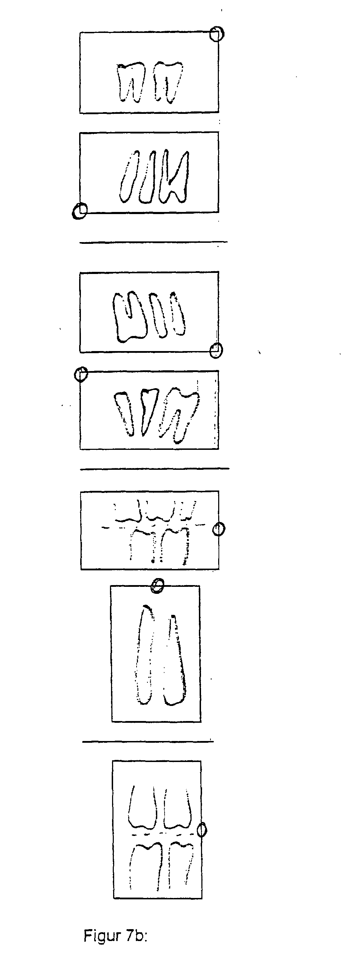

**Figur 7b** zeigt die Anwendung der Grundpositionierungen am Beispiel intraoraler Radiologie.

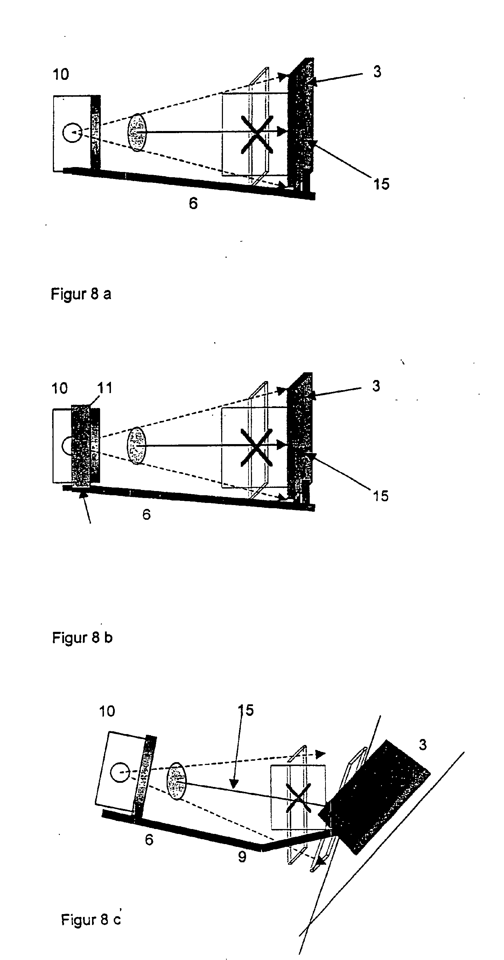

**Figur 8a,8b und 8c** zeigen Aufnahmetechnologien insbesondere in der Zahnmedizin.
Rechtwinkeltechnik:
Hier soll der Zentralstrahl (Röntgen) 15 in der Mitte des Aufnahmemediums 3 (Bildebene) auftreffen und zwar lotrecht. Diese Technik kann durch optische Kontrolle, besser jedoch unter Zuhilfenahme eines Sensorhalters, eingesetzt werden.
Der Röntgenstrahl wird mit Hilfe des Sensorhalters, der fix am Strahler 19 (Tubus) befestigt ist, lotrecht auf die Bildebene geführt.
Bei der Rechtwinkeltechnik ist der Halter mit Film/ Sensor dabei starr mit dem Tubus des Röntgengerätes verbunden.
Paralleltechnik
Der Sensor wird hierbei parallel zum Objekt gestellt. Der Zentralstrahl trifft idealer weise auch hier, unter Verwendung eines Visierrings 11, die BildEbene und die Sensor Ebene lotrecht.
Bei der Paralleltechnik wird ein separater Halter mit Visierring und im Dentalbereich mit Aufbissstück verwendet.
Halbwinkeltechik
Bei sehr großen Abweichungen der eigentlich geforderten Parallelität von Objektebene zu Aufnahmeebene verwendet man die Halbwinkeltechnik um einen Fehler so klein wie möglich zu halten.
Der Zentralstrahl trifft dabei lotrecht auf eine gedachte Ebene der Winkelhalbierenden zwischen Objektebene und Bildebene.

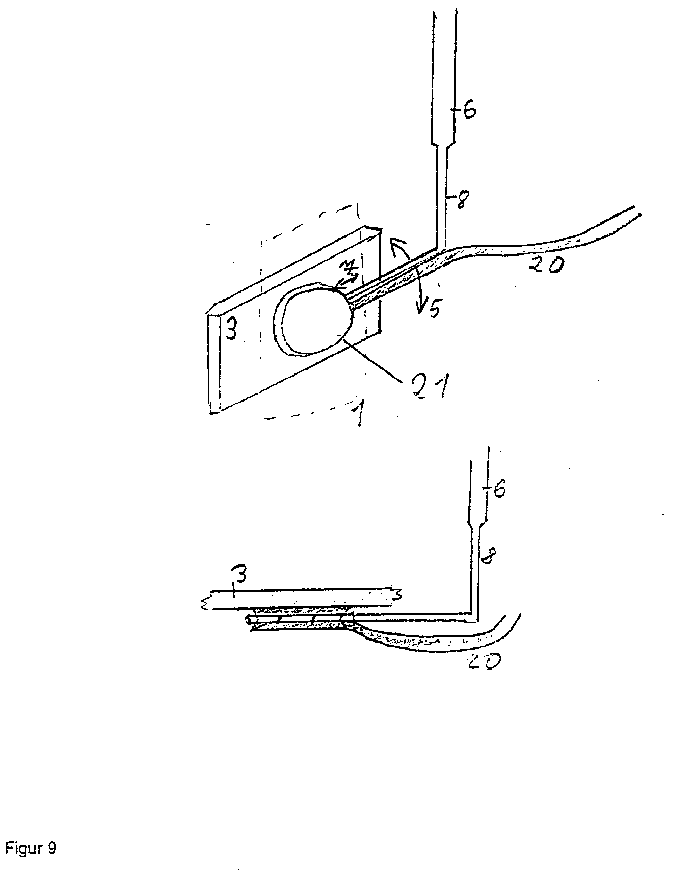

**Figur 9** zeigt ein wiederverwendbares Aufnahmemedium 3, beispielsweise einen Filmsensor mit der Besonderheit der direkten integretion des Haltemechanismus 1 im Aufnahmemedium.
Die Haltestange 8 ist dabei beispielsweise durch einen Klemmmechansimus 21 und oder anderweitig befestigten Haltemechanismus 1 mit dem Aufnahmemedium flexibel (Rotation,Translation) verbunden. Das Verbindungskabel 20 zum Sensor kann damit unterhalb der zueinander beweglichen Teile Aufnahmemedium 3 - Haltemechanismus 1 geführt werden.

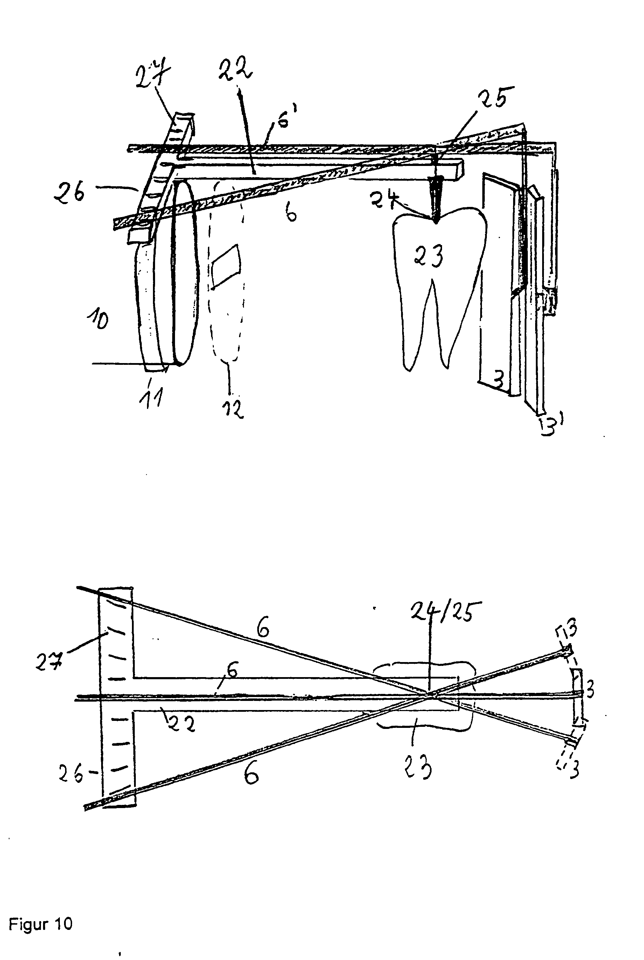

**Figur 10** zeigt die Fixierung 24 der T-Stange 22 am Aufnahmeobjekt 23. Die T-Stange 22 selbst dient zur Fixierung des Strahlers 10 und ist mit dem Visierring 11 am Tubus befestigt.
Die Haltestange 6 ist mit dem Punkt 25 (Verlängerung des Punktes 24) drehbar verbunden. Diese Verbindung erlaubt es die Haltestange 6 um den Punkt 24/25 zu drehen. Das Aufnahmemedium 3 ist wie bereits beschrieben an der Haltestange 6 angebracht Es kann ebenso wie in Figur 4 eine Blende 12 eingesetzt werden. Der Querteil der T-Stange 26 dient zur Rasterung der Haltestange 6 in den Rasterpunkten 27, um hier exakt den Winkel bestimmen zu können. Das Objekt 23 wird aus verschiedenen Winkeln aufnehmbar. Die T-Stange kann auch weggelassen werden. Somit ist die Haltestange 6 direkt mit dem Punkt 24 verbunden. Die Winkeleinstellung entfällt, dies genügt jedoch wenn keine reproduzierbaren Winkel-Aufnahmen erforderlich sind.

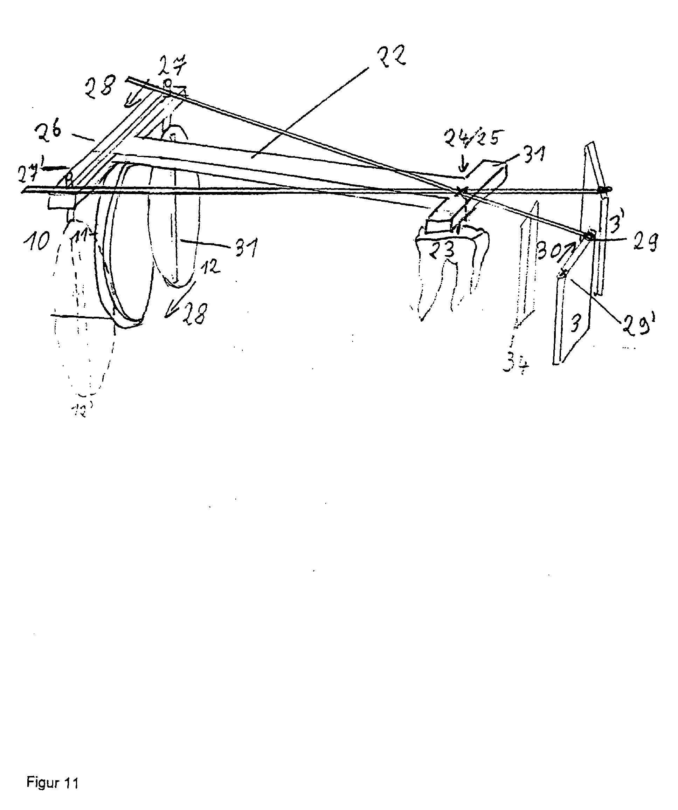

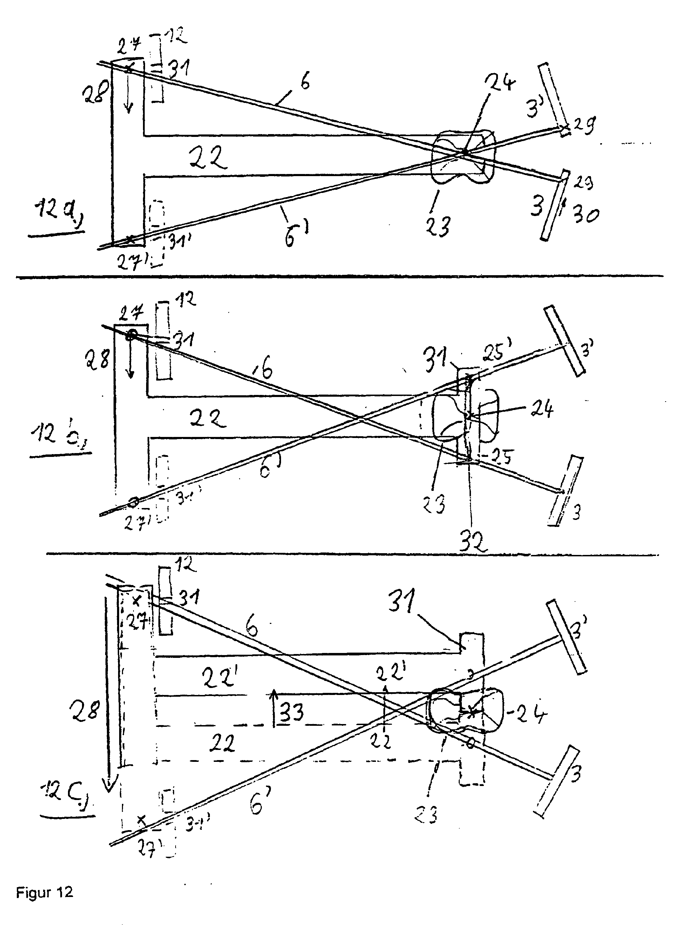

**Figur 11** Mit dem Halter-System kann durch seine universelle und einfache Justierbarkeit eine Schichtaufnahme realisierzt werden. Figur 11 zeigt die Prinzipdarstelllung dieses einfachen Schichtaufnahmegerätes.
Der Grundaufbau ist ähnlich dem in Figur 10. Die einzelnen Fixierungen 27 der Halte-Stange 6 werden durch eine kontinuierliche Bewegung 28 ersetzt. Die Bewegung verläuft von 27 nach 27'. Der Drehpunkt 24/25 bleibt in diesem Ausführungbeispiel wie in Figur 10 auf dem Objekt 23 stehen.
Das Ende 31 des T-Stange 22 dient zur Aufnahme der Verschiebe-Halterungen (siehe Figur 12).
Die Blende 12, die als Schlitzblende 31 (Primärschlitzblende) ausgelegt ist, wird ebenfalls durch die Verschiebung 28 bewegt. Sie verschiebt sich dabei von 12 nach 12'.

Gleichzeitig wird das Aufnahmemedium 3 am Haltestangen-Ende von Punkt 29 mit der Bewegung 30 nach 29' verschoben. Eine Sekundärschlitzblende 34 ist bei nicht parallel anzusehendem Röntgenstrahl (abhängig von der Entfernung Strahler-Primärblende) zu berücksichtigen.
Verschiedene Schichten können durch verdrehen und/oder verschieben der kompletten Konfiguration bezüglich des Objektes 23 erzielt werden.

**Figur 12a-c** zeigen die Bewegungsabläufe bei der Schichtaufnahmetechnik in der Draufsicht unter Verwendung des Halter-Systems. Dargestellt ist die Mechanik des Systems.
Das Grundprinzip der Schichtaufnahme ist mathematisch die Aufintegration von Belichtung (Zeit und Intensität). Der Strahlenaustritt gekennzeichnet durch die Schlitzblende 31 verschoben nach 31' wird durch die Bewegung 28 von oben nach unten von 27 nach 27' verschoben. Diese Verschiebung 28 bleibt in allen 3 folgenden Fällen gleich.
Gleichzeitig wird das Aufnahmemedium 3 durch die zusätzliche Bewegung in 12a, 12b oder 12c, hier gegenläufig, von unten nach oben nach 3' verschoben.
**Figur 12a** zeigt die Verschiebung 30 des Aufnahmemediums am Ende der Haltestange 6 direkt im Punkt 29.
**Figur 12b** zeigt die Verschiebung des Aufnahmemediums (von 3 nach 3'), durch die Verschiebung der Haltestange 6, fixiert im Punkt 25, entlang des Weges 32 nach 25'. Die Fixierung 24 im Objekt 23 bleibt analog (Figur 10/11) bestehen. Das Aufnahmemedium 3 bleibt dabei am Ende der Haltestange mittig fixiert.
**Figur 12c** zeigt die Verschiebung des Aufnahmemediums von 3 nach 3' durch die Paralellverschiebung 33 der T-Stange 22. Das Aufnahmemedium 3 bleibt dabei am Ende der Haltestange mittig fixiert.

Die Kombinatorik der T-Stange mit dem Halter-System ergibt ein mit einfachen Mitteln zu realisierendes Schichtaufnahmegerät. Insbesondere im Zahnbereich ist das in Figur 12b dargestellte System verwendbar.

Um den notwendigen Gleichlauf der beiden Verschiebungen zu erreichen erfolgen diese beispielsweise durch Schrittmotoren.
**Zusammenfassend** betrifft die Erfindung somit ein Halter-System, dass im wesentlichen eine einzige universelle mechanische Vorrichtung zur Abdekkung aller möglichen Positionierungs -Varianten für ein röntgenempfindliches Aufnahmemedium (3) aufweist und im wesentlichen einteilig ausgebildet ist. Mit einem derartigen Halter-System lassen sich alle Positionseinstellungen ohne Wechsel von Verbindungsteilen/Stangen etc. problemlos durchführen. Insbesondere Sensoren können bei verschiedenen aufeinanderfolgenden Aufnahmen im Halter-System fixiert bleiben.
Die hygienischen Bedingungen werden erweitert indem zum einen eine komplette Abdeckung von Aufnahmemedium und Halter-System geschieht zum andern weniger Schutzhüllen benötigt werden.
Ergänzungen des Halter-Systems um einen Mechanismus zur Fixierung sowie einer Verschiebung entlang des Objektes erweitern ein einfaches Röntgensystem um die Möglichkeit exakte Winkeldarstellungen sowie Schichtaufnahmen dieses Objektes durchzuführen.
Bei konventionellen Systemen der Positionierung respektive Halterung des Aufnahmemediums geschieht dies durch Systeme, die sich aus mehreren Teilen zusammensetzen. Diese Teile (verschieden gebogene Stangen, Plastikverbinder und Adapter) werden je nach Notwendigkeit der Positionierung zusammengesteckt und oder verbunden und das Aufnahmemedium daran befestigt.
Der Erfindung liegt die Aufgabe zugrunde, ein Halter-System zu realisieren, das eine einfache und schnelle Positionierung eines Aufnahmemediums ohne Wechsel und aufwendigem Umbau von Halterungen ermöglicht.

## Patentansprüche

1. Halter-System zur Positionierung von Aufnahmemedien bei radiologischen Aufnahmen,
**dadurch gekennzeichnet,**
**dass** das Halter-System im wesentlichen eine einzige universelle mechanische Vorrichtung zur Abdeckung aller möglichen Positionierungs - Varianten für ein röntgenempfindliches Aufnahmemedium (3) aufweist.

2. Halter-System nach Anspruch 1
**dadurch gekennzeichnet,**
**dass** das röntgenempfindliche Aufnahmemedium (3), insbesondere ein Film, Sensor und oder Folie in Lage und Position durch Rotation (5) und / oder Translation (7) positionierbar ist.

3. Halter-System nach einem der Ansprüche 1 oder 2
**dadurch gekennzeichnet,**
**dass** das Halter-System zum befestigen des Aufnahmemediums (3) je nach Größe / Form / Hersteller eine dementsprechend angepasste Halteklammer (2) aufweist, die mit der Haltestange (6) verbunden ist.

4. Halter-System nach einem der Ansprüche 1 bis 3
**dadurch gekennzeichnet,**
**dass** für die verschiedenen Aufnahmepositionen das Halter-System ohne Zerlegung durch einfaches Drehen (5) und oder Verschieben (7) anpassbar ist.

5. Halter-System nach einem der Ansprüche 1 bis 4
**dadurch gekennzeichnet,**
**dass** das Halte-System neben den Grund Positionierungsvarianten durch Zwischenstellungen auch individuell gewünschte Positionen (4a und 4b) zulässt.

6. Halter-System nach einem der Ansprüche 1 bis 5
**dadurch gekennzeichnet,**
**dass** die Halteklammer (2) so ausgebildet ist, dass in der Halteklammer (2) das Aufnahmemedium (3) verschiebbar (7) angebracht ist und /oder Halteklammer (2) und Aufnahmemedium (3) als Einheit verschiebbar sind.

7. Halter-System nach einem der Ansprüche 1 bis 6
**dadurch gekennzeichnet,**
**dass** die Haltestange (6), insbesondere für Aufnahmen in der Zahnmedizin, im Bereich der Zahnreihendurchführung (Figur 6) einen kleinen Querschnitt aufweist, an dem im sensornahem Bereich (8) ein Aufbeissstück (16) angebracht ist (Figur 4), das so ausgelegt ist, das eine geeignete Lagestabilisierung durchgeführt werden kann.

8. Halter-System nach einem der Ansprüche 1 bis 6
**dadurch gekennzeichnet,**
**dass** das Halter-System einen Visierring (11) aufweist, der als Klammer ausgelegt ist und dadurch universell an den Tuben verschiedener Röntgenröhren (10) befestigt werden kann (Figur 4)

9. Halter-System nach einem der Ansprüche 7
**dadurch gekennzeichnet,**
**dass** der Visierring (11) und/oder die Blende (12) zur Strahlenfeldbegrenzung beweglich an dem Tubus (10) befestigt sind, wodurch das Halter-System um den Zentralstrahl (15) drehbar ist.

10. Halter-System nach einem der Ansprüche 8
**dadurch gekennzeichnet,**
**dass** das Halte-System eine Schutzhülle (Hygienehülle) aufweist, die Aufnahmemedium (3), Halteklammer (2) sowie Teile der Haltestange (6) umschließt und diese unter der Schutzhülle verstellbar bleiben (Rotation Translation um den Zentralstrahl).

11. Halter-System nach einem der Ansprüche 9
**dadurch gekennzeichnet,**
**dass** das Aufnahmemedium (3) selbst, den Haltemechanismus (1) oder Teile davon integriert hat (Figur 9).

12. Halter-System nach einem der Ansprüche 10
**dadurch gekennzeichnet,**
**dass** durch die zusätzliche Rotation der Haltestange (6) um den Punkt (24/25) Aufnahmen in einstellbaren Winkeln vom Objekt (23) machbar sind, sowie durch die Fixierpunkte (27) und eine fortlaufende Bewegung (28) diese automatisch angefahren werden können (Figur 10+11).

13. Halter-System nach einem der Ansprüche 11
**dadurch gekennzeichnet,**
**dass** die Haltestange (6) durch die gelagerte Befestigung am Punkt (25) und die Bewegung (28) am Ende (29) einen Kreisbogen beschreibt und durch die Schlitzblende (31) sowie zusätzlich zur translatorischen Bewegung (32 oder 33) eine Verschiebung das Aufnahmemediums (3) die Voraussetzungen für eine Schichtaufnahme bildet (Figur 12).
